# EUROPEAN PATENT APPLICATION

(11) **EP 4 509 605 A1**
(43) Date of publication of application: **19.02.2025**
(21) Application number: 23788364.0
(22) Date of filing: 12.04.2023
(51) Int. Cl.: C12N 15/55, C11D 3/386, C11D 17/06, C11D 17/08, C12N 1/15, C12N 1/19, C12N 1/21, C12N 5/10, C12N 9/20, C12N 15/63

(54) **NOVEL LIPASE**

(30) Priority: 13.04.2022 JP 2022066572
(71) Applicant: Kao Corporation, Tokyo 103-8210 (JP)
(72) Inventor: HIOKI, Takahiro, Wakayama-shi, Wakayama 640-8580 (JP); TERAI, Mika, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2023/014863
(87) International publication number: WO 2023/199946

(57) **Abstract**

Provided is a lipase for which activity inhibition by surfactants is reduced and which exhibits a high cleaning effect. A lipase consisting of the amino acid sequence of SEQ ID NO: 14 or an amino acid sequence having at least 91% identity thereto.

## Description

### Field of the Invention

The present invention relates to a novel lipase.

### Background of the Invention

Lipases are useful for various applications, such as laundry cleaning agents, dishwashing cleaning agents, oil and fat processing, pulp processing, feed, and pharmaceutical intermediate synthesis. In cleaning, lipases hydrolyze triglycerides to produce fatty acids, thereby contributing to the removal of stains including oil.

Current cleaning compositions and cleaning environments contain various components which inhibit the activity and cleaning effect of lipases, and lipases which function under such conditions are required. As a lipase useful for cleaning, *Thermomyces lanuginosus-*derived lipase (hereinafter, TLL) is sold under the product name LIPOLASE (registered trademark). Patent Literature 1 discloses Cedecea sp-16640 strain-derived lipase Lipr139 which has superior cleaning performance in comparison with TLL. Patent Literature 2 discloses a mutant of *Proteus* bacterium-derived lipase (hereinafter, PvLip) which has improved cleaning performance in comparison with one or more reference lipolytic enzymes. Patent Literature 3 discloses metagenomics-derived lipase Lipr138 which has superior cleaning performance in comparison with TLL.

(Patent Literature 1) JP-A-2015-523078
(Patent Literature 2) WO 2020/046613
(Patent Literature 3) JP-A-2015-525248

### Summary of the Invention

The present invention relates to the following 1) to 6):
1) A lipase consisting of the amino acid sequence of SEQ ID NO: 14 or an amino acid sequence having at least 91% identity thereto.
2) A polynucleotide encoding the lipase according to 1).
3) A vector or DNA fragment comprising the polynucleotide according to 2).
4) A transformed cell comprising the vector or DNA fragment according to 3).
5) A cleaning composition comprising the lipase according to 1).
6) A method for cleaning stains, which comprises using the cleaning composition according to 5).

### Brief Description of Drawings

Fig. 1 illustrates the lipase activity of each lipase in surfactant solutions.
Fig. 2 illustrates the lipase activity of each lipase in a surfactant-containing model cleaning liquid.
Fig. 3 illustrates the detergency of each lipase in a surfactant-containing model cleaning liquid.

### Detailed Description of the Invention

All of the patent literatures, non-patent literatures, and other publications cited in the present specification are incorporated herein by reference in their entirety.

In the present specification, "lipase" refers to triacylglycerol lipase (EC3.1.1.3), and means an enzyme group having activity to hydrolyze triglycerides to produce fatty acids. The lipase activity can be determined by measuring the rate of increase in absorbance associated with the release of 4-nitrophenol by the hydrolysis of 4-nitrophenyl octanoate. The specific procedure of measuring the lipase activity is described in detail in the examples provided later.

In the present specification, the identity of amino acid sequences or nucleotide sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

In the present specification, the "operable linkage" between a regulatory region, such as a promoter, and a gene means that the gene and the regulatory region are linked so that the gene can be expressed under the control of the regulatory region. Procedures for the "operable linkage" between the gene and the regulatory region are well known to a person skilled in the art.

In the present specification, the "upstream" and "downstream" relating to a gene refer to the upstream and downstream in the transcription direction of the gene. For example, "a gene located downstream of a promoter" means that the gene is present on the 3' side of the promoter in the DNA sense strand, and the upstream of a gene means a region on the 5' side of the gene in the DNA sense strand.

It is known that the activity of lipases is inhibited by surfactants contained in cleaning compositions. In fact, as verified by the present inventors, known lipases TLL, Lipr139, and PvLip, whose suitability for cleaning is disclosed, were all received large activity inhibition by the coexistence of surfactants. Activity inhibition by surfactants is a major issue common in lipases for cleaning. Further, lipases are often used together with surfactants not only for cleaning applications, but also for industrial applications such as pulp processing, and activity inhibition by surfactants is an issue for the entire industrial lipases. There is a demand for lipases for which activity inhibition by surfactants is reduced and which exhibit a high cleaning effect.

As a result of careful consideration in light of these issues, the present inventors found novel lipase KAL-A8 which has significantly high resistance to activity inhibition by surfactants and exhibits a dramatic cleaning effect.

The lipase of the present invention has significantly high resistance to activity inhibition by surfactants and exhibits an excellent cleaning effect even in the presence of a surfactant.

### <1. Lipase>

The lipase of the present invention is a lipase consisting of the amino acid sequence of SEQ ID NO: 14 or an amino acid sequence having at least 91% identity thereto. The lipase of the present invention has significantly high resistance to lipase activity inhibition by surfactants and exhibits significantly high detergency even in the presence of a surfactant.

Examples of the lipase consisting of an amino acid sequence having at least 91% identity to the amino acid sequence of SEQ ID NO: 14 include lipases consisting of an amino acid sequence having at least 91% identity, specifically 91% or more, preferably 93% or more, more preferably 95% or more, further more preferably 96% or more, further more preferably 97% or more, further more preferably 98% or more, and further more preferably 99% or more identity to the amino acid sequence of SEQ ID NO: 14.

The amino acid sequences having at least 91% identity include amino acid sequences having deletion, insertion, substitution, or addition of one or several amino acids. Examples of the "amino acid sequences having deletion, insertion, substitution, or addition of one or several amino acids" include amino acid sequences having deletion, insertion, substitution, or addition of 1 or more and 30 or fewer, preferably 20 or fewer, more preferably 10 or fewer, and further more preferably 5 or fewer amino acids.

Examples of lipases consisting of an amino acid sequence having at least 91% identity to the amino acid sequence of SEQ ID NO: 14 include artificially generated mutants of the lipase consisting of the amino acid sequence of SEQ ID NO: 14. Such mutants can be produced, for example, by introducing a mutation into a gene encoding the amino acid sequence of SEQ ID NO: 14 by UV irradiation or a known mutagenesis method such as site-directed mutagenesis, expressing the gene with the mutation, and selecting proteins having desired lipase activity. Such a procedure for generating mutants is well known to a person skilled in the art.

The lipase of the present invention has an amino acid sequence which is different from that of conventionally isolated or purified lipases and proteins which have been estimated as triacylglycerol lipases in the NCBI protein sequence database. Examples of conventionally isolated or purified lipases include *Thermomyces lanuginosus-derived* lipase TLL (SEQ ID NO: 18), Cedecea sp.-16640 strain-derived lipase Lipr139 (SEQ ID NO: 2), which is disclosed in Patent Literature 1 mentioned above as a lipase suitable for cleaning, *Proteus* bacterium-derived lipase (hereinafter referred to as PvLip, SEQ ID NO: 4), which is disclosed in Patent Literature 2 mentioned above as a parent enzyme of a lipase mutant group suitable for cleaning, metagenomics-derived lipase Lipr138 (SEQ ID NO: 16), which is disclosed in Patent Literature 3 mentioned above as a lipase suitable for cleaning, and the like. Further, examples of proteins which have been estimated as triacylglycerol lipases in the NCBI protein sequence database include a protein with accession number WP_123598507.1 (hereinafter referred to as PfLip, SEQ ID NO: 6), a protein with accession number WP_115457195.1 (hereinafter referred to as EtLip, SEQ ID NO: 8), a protein with accession number WP_135495634.1 (hereinafter referred to as EspLip, SEQ ID NO: 10), a protein with accession number WP_005161363.1 (hereinafter referred to as YeLip, SEQ ID NO: 12), and the like. The enzymological properties of these proteins registered in this database have not been reported so far. The lipase KAL-A8 of the present invention consisting of the amino acid sequence of SEQ ID NO: 14 has an amino acid sequence identity of 72%, 60%, 59%, 68%, 86%, and 71% to Lipr139, PvLip, PfLip, EtLip, EspLip, and YeLip, respectively.

In a preferred embodiment, the lipase of the present invention is a lipase consisting of the amino acid sequence of SEQ ID NO: 14.

### <2. Method for producing lipase>

The lipase of the present invention can be produced, for example, by expressing a polynucleotide encoding the lipase of the present invention described above. Preferably, the lipase of the present invention can be produced from a transformant into which the polynucleotide encoding the lipase of the present invention is introduced. For example, the polynucleotide encoding the lipase of the present invention, or a vector containing the polynucleotide, is introduced into a host to obtain a transformant, the transformant is then cultured in a suitable culture medium, and as a result, the lipase of the present invention is produced from the polynucleotide encoding the lipase of the present invention introduced into the transformant. The produced lipase can be isolated or purified from the culture to thereby obtain the lipase of the present invention.

The polynucleotide encoding the lipase of the present invention can be a polynucleotide encoding a lipase consisting of the amino acid sequence of SEQ ID NO: 14 or an amino acid sequence having at least 91% identity thereto. Further, the polynucleotide encoding the lipase of the present invention can be in the form of single- or double-stranded DNA, RNA, or an artificial nucleic acid, or may be cDNA or chemically synthesized intron-free DNA.

The polynucleotide encoding the lipase of the present invention can be synthesized chemically or genetically based on the amino acid sequence of the lipase. For example, the polynucleotide can be chemically synthesized based on the amino acid sequence of the lipase of the present invention described above. For the chemical synthesis of the polynucleotide, nucleic acid synthesis contract services (e.g., provided by Medical & Biological Laboratories Co., Ltd., GenScript, and the like) can be used. Further, the synthesized polynucleotide can be amplified by PCR, cloning, or the like.

Alternatively, the polynucleotide encoding the lipase of the present invention can be produced by introducing a mutation into a polynucleotide synthesized by the above procedure by UV irradiation or a known mutagenesis method such as site-directed mutagenesis as described above. For example, the polynucleotide encoding the lipase of the present invention can be obtained by introducing a mutation into the polynucleotide of SEQ ID NO: 13 by a known method, expressing the obtained polynucleotide, examining the lipase activity, and selecting a polynucleotide encoding a protein having desired lipase activity.

Site-directed mutagenesis into the polynucleotide can be performed, for example, by any method, such as an inverse PCR method or an annealing method (edited by Muramatsu et al., "Revised 4th edition, New Handbook of Genetic Engineering", Yodosha Co., Ltd., pp 82-88). If necessary, various commercially available site-specific mutagenesis kits, such as Stratagene's QuickChange II Site-Directed Mutagenesis Kit and QuickChange Multi Site-Directed Mutagenesis Kit, can also be used.

The polynucleotide encoding the lipase of the present invention can be incorporated into a vector. The type of vector to contain the polynucleotide is not particularly limited, and any vector, such as a plasmid, phage, phagemid, cosmid, virus, YAC vector, or shuttle vector, may be used. The vector is not limited, but is preferably a vector which can be amplified in bacteria, preferably *Bacillus* bacteria (e.g., *Bacillus subtilis* or mutant strains thereof), and more preferably an expression vector which can induce the expression of transgenes in *Bacillus* bacteria. Among these, shuttle vectors, which are vectors replicable in *Bacillus* bacteria and any other organisms, can be preferably used in the recombinant production of the lipase of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis;* Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of *Bacillus* bacteria; and the like. Other usable examples include *Escherichia* coli-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence, such as a promoter region for initiating the transcription of the gene, a terminator region, or a secretion signal region for secreting the expressed protein outside the cell, may be operably linked to the upstream of the polynucleotide encoding the lipase of the present invention (i.e., lipase gene of the present invention). The phrase that a gene and a regulatory sequence are "operably linked" herein means that the gene and the regulatory region are arranged so that the gene can be expressed under the control of the regulatory region.

The type of regulatory sequence, such as a promoter region, a terminator region, or a secretion signal region mentioned above, is not particularly limited, and generally used promoters and secretion signal sequences can be appropriately selected depending on the host for introduction. Examples of preferred regulatory sequences that can be incorporated into the vector include the promoter, secretion signal sequence, and the like of the cellulase gene of *Bacllus* sp. KSM-S237 strain.

Alternatively, a marker gene (e.g., a gene resistant to drugs, such as ampicillin, neomycin, kanamycin, and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the lipase of the present invention, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

The transformed cell of the present invention can be obtained by introducing a vector containing the polynucleotide encoding the lipase of the present invention into a host, or by introducing a DNA fragment containing the polynucleotide encoding the lipase of the present invention into the genome of the host.

Examples of the host cells include microorganisms, such as bacteria and filamentous fungi. Examples of bacteria include *Escherichia coli* and bacteria belonging to the genera *Staphylococcus, Enterococcus, Listeria,* and *Bacillus.* Preferred among these are *Escherichia coli* and *Bacillus* bacteria (e.g., *Bacillus subtilis* Marburg No. 168 *(Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include *Trichoderma, Aspergillus, Rhizopus,* and the like.

Methods commonly used in the art, such as the protoplast method and the electroporation method, can be used to introduce the vector into the host. Strains with appropriate introduction are selected using marker gene expression, auxotrophy, and the like as indices, whereby the target transformant into which the vector is introduced can be obtained.

Alternatively, a fragment obtained by linking the polynucleotide encoding the lipase of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the lipase of the present invention is introduced into the genome of the host.

When the thus-obtained transformant into which the polynucleotide encoding the lipase of the present invention, or a vector containing the polynucleotide is introduced, is cultured in a suitable culture medium, the gene encoding the protein on the vector is expressed to produce the lipase of the present invention. The culture medium used for culturing the transformant can be appropriately selected by a person skilled in the art depending on the type of microorganism of the transformant.

Alternatively, the lipase of the present invention may be expressed from the polynucleotide encoding the lipase of the present invention or a transcript thereof using a cell-free translation system. The "cell-free translation system" is such that reagents, such as amino acids, necessary for the protein translation are added to a suspension obtained by mechanically destroying a cell, which serves as the host, to construct an in vitro transcription-translation system or an in vitro translation system.

The lipase of the present invention produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. The protein collected from the culture may be further purified by known means.

### <3. Cleaning composition>

In comparison with known proteins, the thus-obtained lipase of the present invention has significantly higher resistance to lipase activity inhibition by surfactants, and exhibits significantly higher detergency even in the presence of a surfactant.

"Higher resistance" means resistance higher than that of known proteins, specifically lipases consisting of the amino acid sequence of SEQ ID NO: 2, 4, 6, 8, 10, or 12, or proteins that have been estimated as lipases in the NCBI protein sequence database. The resistance to lipase activity inhibition by surfactants can be evaluated by using a method well known in the art. For example, a lipase solution and a surfactant solution are mixed, a 4-nitrophenyl octanoate solution, which serves as a lipase substrate, is added to the mixture, the absorbance change (OD/min) at 405 nm associated with the release of 4-nitrophenol is measured, and the difference ΔOD/min from the blank is determined as the lipase activity value (lipase activity value in the surfactant solution). Further, the lipase activity value when using a buffer in place of the surfactant solution (lipase activity value in the buffer) is determined, and the lipase activity value in the surfactant solution is divided by the lipase activity value in the buffer and then multiplied by 100. The resulting value is determined as the relative activity (%). It can be determined that a higher relative activity (%) indicates higher resistance to lipase activity inhibition by the surfactant.

The lipase of the present invention is a lipase which has the lipase relative activity (%) in an SDS solution (0.1% (w/v)) under the conditions (3) in the examples provided later being preferably 20% or more, and more preferably 30% or more.

The lipase of the present invention is a lipase which has the lipase relative activity (%) in a Triton X-100 solution (0.1% (w/v)) under the conditions (3) in the examples provide later being preferably 50% or more, and more preferably 70% or more.

Further, "high detergency" means detergency higher than that of known proteins, specifically lipases consisting of the amino acid sequence of SEQ ID NO: 2, 4, 6, 8, 10, or 12, or proteins that have been estimated as lipases in the NCBI protein sequence database, for example, an ability to bring about the removal of stains in the washing or cleaning step. The detergency can be evaluated by using a method well known in the art. For example, a lipase-containing cleaning liquid is added to a model stain containing a predetermined indicator substance (e.g., a staining agent highly soluble in fat, such as Sudan III), followed by cleaning treatment under predetermined conditions. A part of the cleaning liquid is taken, the concentration of the indicator substance in the model stain solubilized in the cleaning liquid by the cleaning treatment is measured by, for example, absorbance measurement, and the difference from the blank can be determined as detergency.

The lipase of the present invention is useful as an enzyme to be contained in various cleaning compositions, and particularly useful as an enzyme to be contained in cleaning compositions suitable for low-temperature cleaning.

Examples of the "low temperature" as mentioned herein include 40°C or lower, 35°C or lower, 30°C or lower, and 25°C or lower, and also include 5°C or higher, 10°C or higher, and 15°C or higher. Other examples include from 5 to 40°C, from 10 to 35°C, from 15 to 30°C, and from 15 to 25°C.

The amount of the lipase of the present invention contained in a cleaning composition is not particularly limited as long as the lipase can exhibit activity. For example, the amount of the lipase per kg of the cleaning composition is preferably 0.1 mg or more, more preferably 1 mg or more, and even more preferably 5 mg or more, as well as preferably 5,000 mg or less, more preferably 1,000 mg or less, and even more preferably 500 mg or less. The amount of the lipase is also preferably from 0.1 to 5,000 mg, more preferably from 1 to 1,000 mg, and even more preferably from 5 to 500 mg.

The cleaning composition preferably contains a sulfosuccinic acid ester or a salt thereof, in addition to the lipase of the present invention. Sulfosuccinic acid esters or salts thereof are known as components to be contained in cleaning compositions (e.g., JP-A-2019-182911). The sulfosuccinic acid ester or a salt thereof is preferably a sulfosuccinic acid branched alkyl ester having a branched alkyl group having 9 or more and 12 or less carbon atoms or a salt thereof, more preferably a sulfosuccinic acid branched alkyl ester having a branched alkyl group having 9 or 10 carbon atoms or a salt thereof, and further more preferably a sulfosuccinic acid branched alkyl ester having a branched alkyl group having 10 carbon atoms or a salt thereof. Moreover, the sulfosuccinic acid ester or a salt thereof is preferably a sulfosuccinic acid di-branched alkyl ester having two branched alkyl groups each having 9 or more and 12 or less carbon atoms or a salt thereof, more preferably a sulfosuccinic acid di-branched alkyl ester having two branched alkyl groups each having 9 or 10 carbon atoms or a salt thereof, further more preferably a sulfosuccinic acid di-branched alkyl ester having two branched alkyl groups each having 10 carbon atoms or a salt thereof, and further more preferably bis-(2-propylheptyl) sulfosuccinate or a salt thereof.

Examples of salts include alkali metal salts, alkanolamine salts, and the like, preferably alkali metal salts or alkanolamine salts, more preferably a salt selected from the group consisting of a sodium salt, a potassium salt, a triethanolamine salt, a diethanolamine salt, and a monoethanolamine salt, and further more preferably a sodium salt.

The sulfosuccinic acid ester or a salt thereof is, for example, a compound of the following formula 1. 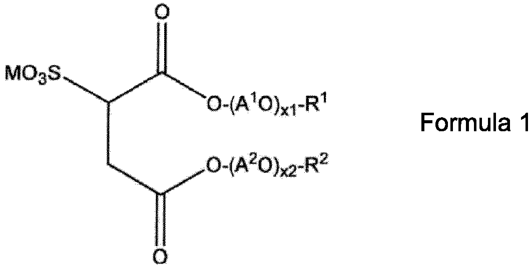 [in formula 1, R¹ and R² are each a branched alkyl group having 9 or more and 12 or less carbon atoms, A¹O and A²O are each an alkyleneoxy group having 2 or more and 4 or less carbon atoms, x1 and x2 are average numbers of mole added and each is a number of 0 or more and 10 or less, and M is a cation.]

In formula 1, R¹ and R² are each preferably a branched alkyl group selected from the group consisting of a branched nonyl group, a branched decyl group, and a branched dodecyl group, and more preferably a branched decyl group. The branched decyl group is preferably a 2-propylheptyl group.

In formula 1, A¹O and A²O are each an alkyleneoxy group having 2 or more and 4 or less carbon atoms, and preferably an alkyleneoxy group having 2 or 3 carbon atoms from the viewpoint of lubricity against water. In formula 1, x1 and x2 are average numbers of mole added of A¹O and A²O, respectively, and each is a number of 0 or more and 10 or less, and preferably 6 or less, more preferably 4 or less, further more preferably 2 or less, and further more preferably 0 from the viewpoint of lubricity against water.

In formula 1, M is a cation. M is preferably a cation other than hydrogen ions. Examples of M include alkali metal ions, such as lithium ions, sodium ions, and potassium ions; alkaline earth metal ions, such as calcium ions and barium ions; organic ammonium ions, such as triethanolammonium ions, diethanolammonium ions, monoethanolammonium ions, trimethylammonium ions, and monomethylammonium ions; and the like.

From the viewpoint of dispersibility in water, M is preferably an alkali metal ion or an alkanol ammonium ion, more preferably a sodium ion, a potassium ion, a triethanolammonium ion, a diethanolammonium ion, or a monoethanolammonium ion, and further more preferably a sodium ion.

The sulfosuccinic acid ester or a salt thereof is preferably a compound of the following formula 1-1. The compound of formula 1-1 is a compound of formula 1 wherein x1 and x2 are each 0. 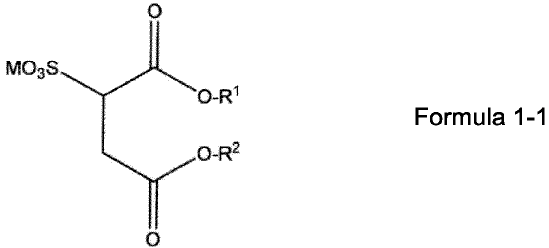 [in formula 1-1, R¹ and R² are each a branched alkyl group having 9 or more and 12 or less carbon atoms, and M is a cation.]

In formula 1-1, specific examples and preferred examples of R¹, R², and M are the same as those in formula 1.

In a preferred embodiment, the sulfosuccinic acid ester or a salt thereof is bis-(2-propylheptyl) sulfosuccinate or a salt thereof.

The amount of the sulfosuccinic acid ester or a salt thereof contained in the cleaning composition is preferably 0.01 mass% or more, more preferably 0.1 mass% or more, and is preferably 2.0 mass% or less, more preferably 1.0 mass% or less. Further, the amount of the sulfosuccinic acid ester or a salt thereof is preferably from 0.01 to 2.0 mass%, and further more preferably from 0.1 to 1.0 mass%.

In the cleaning composition, various enzymes other than the lipase of the present invention can be used in combination. Examples include hydrolases, oxidases, reductases, transferases, lyases, isomerases, ligases, synthetases, and the like. Preferred among these are lipases which are different from the lipase of the present invention, amylases, proteases, cellulases, keratinases, esterases, cutinases, pullulanases, pectinases, mannanases, glucosidases, glucanases, cholesterol oxidases, peroxidases, laccases, and the like; and particularly preferred are proteases, cellulases, amylases, and lipases.

Examples of proteases include commercially available Alcalase, Esperase, Everlase, Savinase, Kannase, and Progress Uno (registered trademarks; Novozymes A/S), PREFERENZ, EFFECTENZ, and EXCELLENZ (registered trademarks; DuPont), Lavergy (registered trademark; BASF), KAP (Kao Corporation), and the like.

Examples of cellulases include Celluclean and Carezyme (registered trademarks; Novozymes A/S); KAC, the alkaline cellulase produced by *Bacillus* sp. KSM-S237 strain described in JP-A-10-313859, and the mutant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation), and the like.

Examples of lipases include Lipolase and Lipex (registered trademarks; Novozymes A/S), and the like.

Known cleaning components can be contained in the cleaning composition, and examples of such known cleaning components include the following.

### (1) Surfactant

The surfactant may be contained in an amount of from 0.5 to 60 mass% in the cleaning composition, and preferably from 10 to 45 mass% particularly in a powder cleaning composition, and from 20 to 90 mass% in a liquid cleaning composition. When the cleaning composition of the present invention is a clothing cleaning agent for laundry or a cleaning agent for an automatic dishwasher, the surfactant is generally contained in an amount of from 1 to 10 mass%, and preferably from 1 to 5 mass%.

Examples of the surfactant used in the cleaning composition include one or a combination of anionic surfactants, nonionic surfactants, amphoteric surfactants, and cationic surfactants, other than the sulfosuccinic acid esters or salts thereof described above, and preferably anionic surfactants and nonionic surfactants.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, α-olefin sulfonate, internal olefin sulfonate, α-sulfo fatty acid salts, α-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfone with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred non-ionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester, and polyoxyethylene polyoxypropylene block polymers. In particular, preferred non-ionic surfactants are polyoxyalkylene alkyl ethers obtained by adding 4 to 20 moles of alkylene oxides, such as ethylene oxide and propylene oxide, to alcohols having from 10 to 18 carbon atoms [an HLB value (calculated by the Griffin method) of from 10.5 to 15.0, and preferably from 11.0 to 14.5].

### (2) Divalent metal ion scavenger

A divalent metal ion scavenger may be contained in an amount of from 0.01 to 50 mass%, and preferably from 5 to 40 mass%. Examples of the divalent metal ion scavenger used in the cleaning composition of the present invention include condensed phosphates, such as tripolyphosphates, pyrophosphates, and orthophosphates; aluminosilicates, such as zeolites; synthetic layered crystalline silicates, nitrilotriacetates, ethylenediaminetetraacetates, citrates, isocitrates, polyacetal carboxylates, and the like. Among these, crystalline aluminosilicates (synthetic zeolites) are particularly preferred. Among A-, X-, and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

An alkali agent may be contained in an amount of from 0.01 to 80 mass%, preferably from 1 to 40 mass%. In the case of powder cleaning agents, examples of alkali agents include alkali metal carbonates, such as sodium carbonate, collectively called dense ash or light ash; and amorphous alkali metal silicates, such as JIS Nos. 1, 2, and 3. These inorganic alkali agents are effective in the formation of the particle skeleton during drying of cleaning agent, and relatively hard cleaning agents having excellent flowability can be obtained. Examples of other alkalis include sodium sesquicarbonate, sodium hydrogencarbonate, and the like. In addition, phosphates, such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-redeposition agent

An anti-redeposition agent may be contained in an amount of from 0.001 to 10 mass%, preferably from 1 to 5 mass%. Examples of the anti-redeposition agent used in the cleaning composition of the present invention include polyethylene glycol, carboxylic acid-based polymers, polyvinyl alcohol, polyvinylpyrrolidone, and the like. Among these, carboxylic acid-based polymers have the function of scavenging metal ions and the ability to disperse solid particle stains from the clothing into the laundry bath, as well as the anti-redeposition ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavenging, dispersion, and anti-redeposition ability.

### (5) Bleaching agent

A bleaching agent, such as hydrogen peroxide or percarbonate, may be preferably contained in an amount of from 1 to 10 mass%. When the bleaching agent is used, tetraacetylethylenediamine (TAED) or the bleach activator described in JP-A-6-316700 can be contained in an amount of from 0.01 to 10 mass%.

### (6) Fluorescent agent

Examples of a fluorescent agent used in the cleaning composition include biphenyl-type fluorescent agents (e.g., Tinopal CBS-X and the like) and stilbene-type fluorescent agents (e.g., a DM-type fluorescent dye and the like). The fluorescent agent may be preferably contained in an amount of from 0.001 to 2 mass%.

### (7) Other components

The cleaning composition may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleaning composition can be produced in accordance with a standard method by combining the lipase of the present invention obtained by the above method and the known cleaning components mentioned above. The form of the cleaning agent can be selected depending on the application. For example, the cleaning agent can be in the form of liquid, powder, granules, paste, solids, or the like.

The thus-obtained cleaning composition can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleaning composition is suitable for use at 40°C or lower, 35°C or lower, 30°C or lower, or 25°C or lower, and at 5°C or higher, 10°C or higher, or 15°C or higher. The cleaning composition is also suitable for use at from 5 to 40°C, from 10 to 35°C, from 15 to 30°C, or from 15 to 25°C. Examples of preferred use modes include use in laundry cleaning at low temperatures (from 15 to 30°C) and use in cleaning by automatic dishwashers at low temperatures (from 15 to 30°C).

Due to the use of the cleaning composition of the present invention, items to be cleaned (e.g., clothing, dishes, hard surfaces, drainage pipes, dentures, medical instrument, and the like) which require the removal of stains can be cleaned; that is, stains can be removed. Such a cleaning method includes bringing an item to be cleaned which requires the removal of stains into contact with the cleaning composition of the present invention. Preferably, the stains are sebum stains or stains containing food-derived oils and fats.

In the cleaning method of the present invention, in order to bring an item to be cleaned into contact with the cleaning composition, the item to be cleaned may be immersed in water in which the cleaning composition is dissolved, or the cleaning composition may be directly applied to the item to be cleaned. In the method of the present invention, the item to be cleaned after immersion or application of the cleaning composition may be further washed by hand or in a washing machine, which is not always necessary.

Regarding the embodiments described above, the present invention further discloses the following aspects.
<1> A lipase consisting of the amino acid sequence of SEQ ID NO: 14 or an amino acid sequence having at least 91% identity thereto.
<2> The lipase according to <1>, which consists of the amino acid sequence of SEQ ID NO: 14.
<3> A polynucleotide encoding the lipase according to <1> or <2>.
<4> A vector or DNA fragment comprising the polynucleotide according to <3>.
<5> A transformed cell comprising the vector or DNA fragment according to <4>.
<6> The transformed cell according to <5>, which is a microorganism.
<7> The transformed cell according to <5> or <6>, which is *Escherichia coli* or a *Bacillus* bacterium.
<8> A cleaning composition comprising the lipase according to <1> or <2>.
<9> The cleaning composition according to <8>, further comprising a sulfosuccinic acid ester or a salt thereof, preferably a sulfosuccinic acid branched alkyl ester having a branched alkyl group having 9 or more and 12 or less carbon atoms or a salt thereof, more preferably a sulfosuccinic acid branched alkyl ester having a branched alkyl group having 9 or 10 carbon atoms or a salt thereof, and further more preferably a sulfosuccinic acid branched alkyl ester having a branched alkyl group having 10 carbon atoms or a salt thereof.
<10> The cleaning composition according to <8>, further comprising a sulfosuccinic acid diester or a salt thereof, preferably a sulfosuccinic acid di-branched alkyl ester having two branched alkyl groups each having 9 or more and 12 or less carbon atoms or a salt thereof, more preferably a sulfosuccinic acid di-branched alkyl ester having two branched alkyl groups each having 9 or 10 carbon atoms or a salt thereof, further more preferably a sulfosuccinic acid di-branched alkyl ester having two branched alkyl groups each having 10 carbon atoms or a salt thereof, and further more preferably bis-(2-propylheptyl) sulfosuccinate or a salt thereof.
<11> The cleaning composition according to any one of <8> to <10>, which is a clothing cleaning agent or a dishwashing cleaning agent.
<12> The cleaning composition according to any one of <8> to <11>, which is a powder or a liquid.
<13> The cleaning composition according to any one of <8> to <12>, which is used at a low temperature.
<14> The cleaning composition according to <13>, which is used at 40°C or lower, 35°C or lower, 30°C or lower, or 25°C or lower, and at 5°C or higher, 10°C or higher, or 15°C or higher, or at from 5 to 40°C, from 10 to 35°C, from 15 to 30°C, or from 15 to 25°C.
<15> A method for cleaning stains, which comprises using the cleaning composition according to any one of <8> to <14>.
<16> The method according to <15>, which comprises bringing an item to be cleaned into contact with the cleaning composition according to any one of <8> to <14>.
<17> The method according to <15> or <16>, wherein the stains are sebum stains or stains containing food-derived oils and fats.
<18> Use of a lipase consisting of the amino acid sequence of SEQ ID NO: 14 or an amino acid sequence having at least 91% identity thereto for producing a cleaning composition.
<19> The use according to <18>, wherein the lipase is a lipase consisting of the amino acid sequence of SEQ ID NO: 14.
<20> The use according to <18> or <19>, wherein the cleaning composition is a clothing cleaning agent or a dishwashing cleaning agent.
<21> The use according to any one of <18> to <20>, wherein the cleaning composition is a powder or a liquid.
<22> The use according to any one of <18> to <21>, wherein the cleaning composition is used at a low temperature.
<23> The use according to <22>, wherein the cleaning composition is used at 40°C or lower, 35°C or lower, 30°C or lower, or 25°C or lower, and at 5°C or higher, 10°C or higher, or 15°C or higher, or at from 5 to 40°C, from 10 to 35°C, from 15 to 30°C, or from 15 to 25°C.
<24> Use of a lipase consisting of the amino acid sequence of SEQ ID NO: 14 or an amino acid sequence having at least 91% identity thereto for cleaning stains.
<25> The use according to <24>, wherein the lipase is a lipase consisting of the amino acid sequence of SEQ ID NO: 14.
<26> The use according to <24> or <25>, wherein the stains are sebum stains or stains containing food-derived oils and fats.
<27> The use according to any one of <24> to <26>, wherein the cleaning is cleaning at a low temperature.
<28> The use according to <27>, wherein the cleaning is cleaning at 40°C or lower, 35°C or lower, 30°C or lower, or 25°C or lower, and at 5°C or higher, 10°C or higher, or 15°C or higher, or cleaning at from 5 to 40°C, from 10 to 35°C, from 15 to 30°C, or from 15 to 25°C.

### Examples

The present invention is described in more detail below based on examples; however, the present invention is not limited thereto.

### (1) Construction of lipase expression plasmids

Lipase expression plasmids were constructed using the VHH expression plasmid of SEQ ID NO: 26 containing a *Bacillus subtilis* spoVG gene-derived promoter described in WO2021/153129 as a template. The plasmids were constructed by transferring each lipase gene to the full length of ORF containing the VHH gene of the above plasmid by the In-Fusion reaction. Plasmids pHY-Lipr139, pHY-PvLip, pHY-PfLip, pHY-EtLip, pHY-EspLip, pHY-YeLip, pHY-KAL-A8, and pHY-Lipr138 were constructed from artificially synthesized lipase genes Lipr139, PvLip, PfLip, EtLip, EspLip, YeLip, KAL-A8, and Lipr138 (the polynucleotides of respective SEQ ID NOs: 1, 3, 5, 7, 9, 11, 13, and 15, which respectively encode the amino acid sequences of SEQ ID NOs: 2, 4, 6, 8, 10, 12, 14, and 16), respectively. Artificially synthesized lipase TLL gene (the polynucleotide of SEQ ID NO: 17, which encodes the amino acid sequence of SEQ ID NO: 18) with *Bacillus subtilis* amyE-derived signal sequence (the polynucleotide of SEQ ID NO: 19, which encodes the amino acid sequence of SEQ ID NO: 20) linked to the N-terminus was transferred to the full length of ORF containing the Lipr139 gene of the plasmid pHY-Lipr139 by the In-Fusion reaction to construct pHY-amyEsig-TLL.

### (2) Preparation of lipase solutions

The lipase expression plasmids were each introduced into *Bacillus subtilis* strain by the protoplast method, and cultured in 2×L-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline; % denotes (w/v)%) at 30°C for 2 days. Then, the lipase-containing culture supernatant was collected by centrifugation. The culture supernatant was replaced with 10 mM Tris-HCl+0.01% Triton-X100 (pH: 7.0) buffer by dialysis, and the lipase concentration was quantified from the band intensity of SDS-PAGE.

### (3) Measurement of activity in surfactant solution

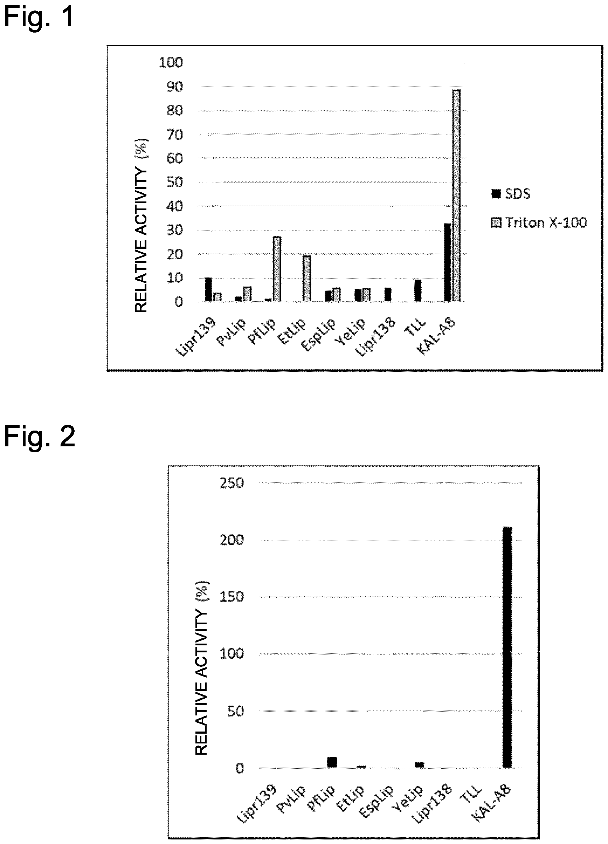

4-Nitrophenyl octanoate (SIGMA) was used as a substrate. The lipase activity can be determined by measuring the rate of increase in absorbance associated with the release of 4-nitrophenol by the action of the lipase. 20 mM 4-nitrophenyl octanoate in 20 mM Tris-HCl (pH: 7.0) was used as a substrate solution. The surfactant solution used was prepared by adding 0.1% (w/v) SDS or Triton X-100 to 20 mM Tris-HCl (pH: 7.0). 5 µL of the lipase solution adjusted to 2 ppm and 100 µL of the surfactant solution were mixed in each well of a 96-well assay plate, 10 µL of the substrate solution was added thereto, and the absorbance change (OD/min) at 405 nm was measured at 30°C. The difference ΔOD/min from the blank (enzyme-free sample) was used as the activity value. The activity value of each lipase in the surfactant solution was divided by the activity value when using 20 mM Tris-HCl (pH: 7.0) (activity in the buffer) in place of the surfactant solution, and then multiplied by 100. The resulting value was determined as the relative activity (%) (Fig. 1).

The activity of TLL, and Lipr139, PvLip, and Lipr138, whose suitability for cleaning is disclosed in Patent Literatures 1, 2, and 3, as well as PfLip, EtLip, EspLip, and YeLip, which are naturally existing lipase sequences, was significantly inhibited in the presence of SDS and Triton X-100. In comparison with these, the novel lipase KAL-A8 showed high resistance to activity inhibition by SDS and Triton X-100.

### (4) Measurement of activity in model cleaning liquid

20 mM 4-nitrophenyl octanoate in 20 mM Tris-HCl (pH: 7.0) was used as a substrate solution. The aqueous medium of Table 1 was used as a model cleaning liquid. 2 µL of the lipase solution adjusted to 4 ppm and 100 µL of a test liquid (model cleaning liquid or 20 mM Tris-HCl (pH: 7.0)) were mixed in each well of a 96-well assay plate, 10 µL of the substrate solution was added thereto, and the absorbance change (OD/min) at 405 nm was measured at 30°C. The difference ΔOD/min from the blank (enzyme-free sample) was determined. 10 µL of 20 mM Tris-HCl (pH: 7.0) containing from 31 to 500 µM of 4-nitrophenol was mixed with 100 µL of each test liquid, the absorbance at 405 nm was measured, and a calibration curve was created. From the calibration curve of each test liquid and the ΔOD/min value, the release rate (µM/min) of 4-nitrophenol per minute was calculated as the activity value.

The activity value of each lipase when using the model cleaning liquid as the test liquid was divided by the activity value when using 20 mM Tris-HCl (pH: 7.0) as the test liquid (activity in the buffer), and then multiplied by 100. The resulting value was determined as the relative activity (%) (Fig. 2).

The activity of TLL, and Lipr139, PvLip, and Lipr138, whose suitability for cleaning is disclosed in Patent Literatures 1, 2, and 3, as well as PfLip, EtLip, EspLip, and YeLip, which are naturally existing lipase sequences, was significantly inhibited in the model cleaning liquid. On the other hand, the novel lipase KAL-A8 showed high resistance to activity inhibition in the model cleaning liquid.

**[Table 1]**

| Component contained | mass% |
|---|---|
| Sulfosuccinic acid branched alkyl ester | 0.2 |
| Betaine | 2 |
| Butyl diglycol | 6.5 |
| Water | Balance |
| Total | 100 |

- Sulfosuccinic acid branched alkyl ester: sodium bis-(2-propylheptyl) sulfosuccinate
- Betaine: lauryl(2-hydroxy-3-sulfopropyl)dimethyl betaine, Amphitol 20HD, manufactured by Kao Corporation

### (5) Cleaning evaluation in model cleaning liquid

Beef tallow (SIGMA, 03-0660) and rapeseed oil (SIGMA, 23-0450) were mixed at a weight ratio of 9:1, and dissolved in 3 times the amount of chloroform, followed by coloring with 0.2 wt% Sudan III, and the resultant was used as a model stain. 10 µL of the model stain was added dropwise to the bottom of each well of a 96-deep-well polypropylene plate, and chloroform was evaporated and dried to prepare a stain plate.

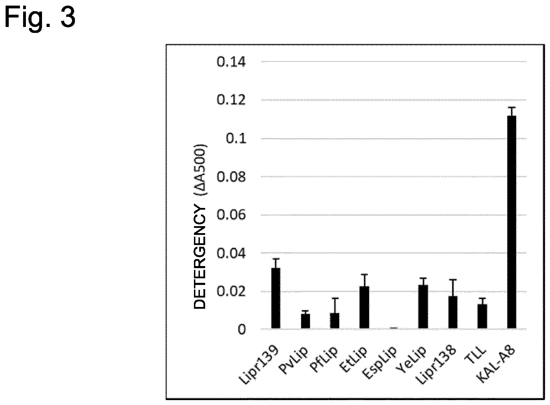

The cleaning liquid used was prepared by adding 1/100 volume of 240 ppm lipase solution to the model cleaning liquid shown in Table 1. 300 µL of the cleaning liquid was added slowly to each well of the stain plate, and allowed to stand at room temperature (about 22°C) for 15 minutes to perform immersion cleaning. 100 µL of the cleaning liquid was taken from each well so as not to contact the stain on the bottom and transferred to a new 96-well plate. In order to quantify Sudan III in the model stain solubilized in the cleaning liquid by immersion cleaning, the absorbance at 500 nm (A500) was measured. A value ΔA500 obtained by subtracting A500 before cleaning from A500 after cleaning corresponds to the amount of oil released in the cleaning liquid, and can be used as an index for detergency. The detergency ΔA500 of each lipase is shown in Fig. 3.

Lipr139, whose suitability for cleaning is disclosed in Patent Literature 1, showed higher detergency in the model cleaning liquid in comparison with TLL, Lipr138, PvLip, PfLip, EtLip, EspLip, and YeLip. On the other hand, the novel lipase KAL-A8 showed significantly higher detergency in the model cleaning liquid in comparison with the other lipases, including Lipr139.

## Claims

1. A lipase consisting of the amino acid sequence of SEQ ID NO: 14 or an amino acid sequence having at least 91% identity thereto.

2. A polynucleotide encoding the lipase according to claim 1.

3. A vector or DNA fragment comprising the polynucleotide according to claim 2.

4. A transformed cell comprising the vector or DNA fragment according to claim 3.

5. The transformed cell according to claim 4, which is a microorganism.

6. A cleaning composition comprising the lipase according to claim 1.

7. The cleaning composition according to claim 6, which is a clothing cleaning agent or a dishwashing cleaning agent.

8. The cleaning composition according to claim 7, which is a powder or a liquid.

9. The cleaning composition according to claim 7 or 8, which is used at a low temperature.

10. The cleaning composition according to claim 9, which is used at a temperature of from 5 to 40°C.

11. A method for cleaning stains, which comprises using the cleaning composition according to any one of claims 6 to 10.
